# EUROPÄISCHE PATENTSCHRIFT

(11) **EP 4 201 365 B1**
(45) Veröffentlichungstag und Bekanntmachung des Hinweises auf die Patenterteilung: **15.05.2024**
(21) Anmeldenummer: 22208692.8
(22) Anmeldetag: 22.11.2022
(51) Int. Cl.: A61C 1/00

(54) **BEHANDLUNGSEINHEIT MIT MESSEINHEIT ZUR BESTIMMUNG VON KEIMREDUKTIONSMITTELN**
TREATMENT UNIT WITH MEASURING UNIT FOR DETERMINING GERM REDUCING AGENTS
UNITÉ DE TRAITEMENT DOTÉE D'UNE UNITÉ DE MESURE POUR DÉTERMINER DES AGENTS RÉDUCTEURS DE GERMINATION

(30) Priorität: 22.12.2021 AT 510392021
(43) Veröffentlichungstag der Anmeldung: 28.06.2023
(73) Patentinhaber: Pregenzer, Lukas, 6414 Mieming (AT)
(72) Erfinder: Pregenzer, Lukas, 6414 Mieming (AT)
(74) Vertreter: Frick, Robert

(56) Entgegenhaltungen:
- EP-A1- 0 428 031
- WO-A1-2019/161334
- WO-A1-2019/165552
- WO-A1-2021/092127
- US-B1- 7 056 472
- O'DONNELL MARY J ET AL: "Management of dental unit waterline biofilms in the 21st century", FUTURE MICROBIOLOGY, Bd. 6, Nr. 10, 1. Oktober 2011 (2011-10-01), Seiten 1209-1226, XP093006872, GB ISSN: 1746-0913, DOI: 10.2217/fmb.11.104

## Beschreibung

Die Erfindung betrifft eine medizinische Behandlungseinheit, insbesondere eine zahnärztliche Behandlungseinheit mit einem wasserführenden System und mit einer Messeinheit zur quantitativen Bestimmung eines Gehalts an Keimreduktionsmitteln in Wasser, das in dem System geführt wird.

Um Wasser aus dem Trinkwassernetz für die Verwendung in einer medizinischen und vorzugsweise zahnärztlichen Behandlungseinheit aufzubereiten, werden aktuell Wasseraufbereitungsanlagen vor oder in der Behandlungseinheit installiert. Diese Anlagen greifen auf bekannte Verfahren zurück, um eine Keimbelastung innerhalb des Wassers zu reduzieren. Diese bekannten Verfahren umfassen eine Beimischung von desinfizierenden Chemikalien wie beispielsweise Wasserstoffperoxid, ein Silbersalz wie Silbernitrat, Phenoxyethanol, Hypochlorsäure oder ein Hypochlorit, Beaufschlagung mit beispielsweise Ozon oder Chlorgas, oder die elektrolytische Spaltung von Wasserinhaltsstoffen wie beispielsweise Natriumchlorid Kochsalz zur Gewinnung von Hypochlorsäure oder einem Hypochlorit. Diese Prozesse zum Versetzen des Wassers mit einem Keimreduktionsmittel werden so gestaltet, dass eine genaue Dosierung angenommen werden kann. Um die genaue Dosierung aber zu bestätigen, wird herstellerseitig meist eine wöchentliche Überprüfung mittels Teststreifen empfohlen (anwendbar etwa bei Wasserstoffperoxid, Chlor, Hypochlorsäure oder Hypochloriden). Weiterhin ist bekannt, zyklisch bakteriologische Abstriche durch zertifizierte Labore durchzuführen.

Gattungsgemäße medizinische Behandlungseinheiten werden in der WO 2019/161334 A1, der WO 2021/092127 A1, der EP 0 428 031 A1, der US 7,056,472 B1 und in O'Donnell, M. J., et al., "Management of dental unit waterline biofilms in the 21st century", Future Microbiol. (2011) 6(10), 1209-1226 offenbart. Ein Verfahren zur Entkeimung von wasserführenden Rohren im Allgemeinen ist in der WO 2019/165552 A1 offenbart.

Da diese Überprüfungsmethoden jedoch immer nur eine Momentaufnahme darstellen und auch Fehler bei der Messung bzw. Probenahme durch den Anwender passieren können, können sie nicht als kontinuierliche und verifizierbare Überprüfung angesehen werden. Ferner kann auf deren Grundlage keine kontinuierliche Einstellung der Menge der zugesetzten Keimreduktionsmittel vorgenommen werden.

Aufgabe der Erfindung ist vor diesem Hintergrund, eine Lösung bereitzustellen, um die Patientensicherheit und Rechtssicherheit in Bezug auf die mikrobiologische Belastung des Behandlungswassers erhöhen zu können.

Vor diesem Hintergrund betrifft die Erfindung eine Medizinische, insbesondere zahnärztliche Behandlungseinheit mit einem wasserführenden System, einer Steuereinheit und einer Wasseraufbereitungsanlage zum Zusatz eines Keimreduktionsmittels in das Behandlungswasser, das in dem System geführt wird. Erfindungsgemäß ist vorgesehen, dass die Behandlungseinheit ferner eine im wasserführenden System stromabwärts der Wasseraufbereitungsanlage angeordnete Messeinheit zur Bestimmung der Konzentration des Keimreduktionsmittels im Behandlungswasser ausweist.

Die Steuereinheit ist mit der Messeinheit und der Wasseraufbereitungsanlage signalverbunden.

Anhand der Messeinheit kann eine kontinuierliche oder zumindest viel engmaschigere Messung der Konzentration des Keimreduktionsmittels vorgenommen werden. Diese Kontrolle genügt allen Anforderungen, erhöht die Patientensicherheit, und wäre auch geeignet, den Arzt bzw. Zahnarzt rechtlich zu entlasten, sofern es beispielsweise zu einem von außen, durch Kontamination der Wasserversorgung, induzierten Legionellenbefall kommt.

Bei dem Keimreduktionsmittel kann es sich beispielsweise um Wasserstoffperoxid, ein Silbersalz wie Silbernitrat, Phenoxyethanol, Chlor, Hypochlorige Säure, ein Hypochlorit, ein Amin, eine quaternäre Amoniumverbindung, Ozon, Iod oder eine Kombination daraus handeln. Bevorzugt kann eine Kombination aus Wasserstoffperoxid und einem Silbersalz, Phenoxyethanol oder beidem sein.

Das oder die Keimreduktionsmittel können in der Wasseraufbereitungsanlage beispielsweise durch Beimischung als feste oder flüssige Chemikalie, durch Beaufschlagung als gasförmige Chemikalie, oder durch in situ Herstellung durch etwa elektrolytische Spaltung von Wasserinhaltsstoffen zugesetzt werden.

Gemäß der Erfindung vorgesehen ist eine indirekte Bestimmung der Konzentration eines möglicherweise nicht oder schwer detektierbaren Keimreduktionsmittels, indem in der Wasseraufbereitungsanlage ein detektierbarer Beistoff, der selbst nicht keimreduzierend wirkt, in einer zum Keimreduktionsmittel proportionalen Menge zugegeben wird, und die Messeinheit die Konzentration des Keimreduktionsmittels im Behandlungswasser unter Messung der Konzentration des Beistoffs bestimmt.

In einer Ausführungsform umfasst die Messeinheit ein UV-VIS-Spektrometer zur beispielsweise Transmissions- oder Extinktionsmessung. Diese Messeinheit lässt sich sehr einfach und kostengünstig implementieren und kann bei geeigneter Wahl des Wellenlängenbereichs eine quantitative Bestimmung UV-aktiver oder farbiger Keimreduktionsmittel (z.B. 2-Phenoxyethanol, HOCI, etc.) oder Beistoffe vornehmen. Im Falle der Verwendung einer derartigen Messeinheit kann bevorzugt sein, dass die Messstrecke zwischen 0.5 mm und 20 mm beträgt. Bevorzugte Bereiche liegen zwischen 2 mm und 10 mm. Eine kleine Messtrecke begünstigt in manchen Fällen die Genauigkeit der Messung auch bei geringer Lichtintensität, kann allerdings apparativ schwierig zu realisieren sein.

In einer Ausführungsform umfasst die Messeinheit ein (N)IR-Spektrometer. Mit einer derartigen Messeinheit lassen sich Keimreduktionsmittel wie beispielsweise Wasserstoffperoxid bestimmen.

In einer Ausführungsform umfasst die Messeinheit einen elektrochemischen Sensor. Elektrochemische Sensoren eignen sich beispielsweise zur Bestimmung von Ozon.

Die Messeinheit kann auch Kombinationen der oben dargestellten Sensoren umfassen.

Da beispielsweise ein IR-Spektrum und insbesondere die für eine quantitative Messung entscheidende Intensität der Peaks in der Regel mit der Temperatur variiert, aber auch andere Messungen durch die Temperatur beeinflusst sein können, kann die Messeinheit auch einen Temperatursensor und/oder eine Temperiereinheit umfassen. Da beispielsweise elektrochemische Messungen, aber auch andere Messungen, mit dem pH-Wert variieren können, kann die Messeinheit auch einen pH-Sensor umfassen.

In einer Ausführungsform umfasst die Behandlungseinheit eine mit der Steuereinheit signalverbundene Leseeinrichtung zum Einlesen von Codes, insbesondere von RFID- oder QR-Codes aufweist, mit denen Behälter von Keimreduktionsmitteln bzw. Beistoffen ersehen sein können.

Die Behandlungseinheit kann ferner eine mit dieser Steuereinheit oder direkt der Messeinheit signalverbundene Schnittstelle zur drahtgebundenen oder vorzugsweise drahtlosen Kommunikation aufweisen. Anhand dieser Verbindungen kann eine Ausgabe von Messwerten (z. B. über CAN oder I2C), eine unidirektionale oder bidirektionale Datenübertragung über ein Netzwerk (z.B. über WiFi oder BT), und/oder eine Kommunikation mit einem RFID- oder QR-System, z.B. zur Identifikation von zur Aufbereitung eingesetzten Keimreduktionsmitteln anhand eines mittels der Leseeinrichtung eingelesenen Codes erfolgen.

Die Steuereinheit kann ausgebildet sein, um Keimreduktionsmittel oder Beistoffe mit bekannter Identität und Konzentration durch eine Codierung anhand der Leseeinheit automatisch zu erkennen, und benötigte Daten gegebenenfalls über die Schnittstelle aus beispielsweise dem Internet zu beziehen. Auf diese Weise kann die Steuereinheit beispielsweise eine automatische Kalibration der Sensoren oder eine automatische Einordnung der Messergebnisse vornehmen.

In einer Ausführungsform ist die Steuereinheit ausgebildet, die Zusatzmenge der Keimreduktionsmittel in der Wasseraufbereitungsanlage auf der Grundlage der Messergebnisse der Messeinheit automatisch zu verändern. Diese Art der Rückkopplung erlaubt eine optimierte Einstellung der Konzentration und mithin eine optimierte Patientensicherheit bei möglichst geringer Keimreduktionsmittelbelastung und möglichst geringem Keimreduktionsmittelverbrauch.

In einer Ausführungsform kann die Behandlungseinheit zwei im wasserführenden System hintereinander geschaltete Messeinheiten aufweisen, um einen etwaigen Konzentrationsabfall des Keimreduktionsmittels in einem Abschnitt des wasserführenden Systems messen und dadurch Rückschlüsse auf das etwaige Vorhandensein von das Keimreduktionsmittel "verbrauchenden" Keimen in dem betreffenden Abschnitt ziehen zu können. Sollte ein solcher Konzentrationsabfall beispielsweise bei Verwendung von Wasserstoffperoxid als Keimreduktionsmittel gemessen werden kann man zurückführen, dass eine Reduktion des Wasserstoffperoxids auf Grund der Oxidation organischer Verbindungen (Biofilm, Bakterien) zwischen den Messpunkten stattgefunden hat. Somit kann der Wert für die Konzentrationsdifferenz als Indikator für den Grad der Kontamination herangezogen werden.

Die Anordnung der Messeinheiten zum Zwecke einer derartigen Zwei-Punkte-Messung kann beispielsweise so sein, dass eine erste Messeinheit kurz hinter (stromabwärts) der Wasseraufbereitungsanlage angeordnet ist, und eine zweite Messeinheit entweder nahe dem Ende des wasserführenden Systems, im Falle einer zahnärztlichen Behandlungseinheit beispielsweise nahe dem Anschluss für ein Handwerkzeug, oder jedenfalls nach einem Abschnitt des wasserführenden Systems, in den Abwärme anderer Komponenten der Behandlungseinheit (z.B. Leistungselektronik, Stellmotoren) eingeleitet wird und in dem ein Bakterienwachstum daher begünstigt sein kann.

In einer Ausführungsform kann das Messergebnis als Grundlage für eine temperaturbedinge Prognose von Wachstumsbedingungen von Mikroorganismen innerhalb der Behandlungseinheit verwendet werden. Sowohl bei einer Ein-Punkt- als auch bei einer Zwei-Punkt-Messung kann in Kombination mit einem an den jeweiligen Messeinheiten verbauten, oder auch andernorts im wasserführenden System angeordneten Temperatursensor festgestellt werden, ob bei bestimmten Temperaturen ein günstiges Keimwachstum stattfinden kann. Diese Information kann im Anschluss dazu verwendet werden, um die Konzentration der keimreduzierenden Komponenten an die Umgebungsbedingungen anzupassen, z.B. Erhöhung der Konzentration unter Rückgriff auf eine Information zur Wassertemperatur.

Die oder jede Messeinheit kann im wasserführenden System an der Hauptleitung oder einer Bypass-Leitung angeordnet sein. Bei Anordnung an einer Bypass-Leitung kann über Umschaltventile ein Batch-Betrieb mit periodischer Messung realisiert werden. Bei Anordnung an der Hauptleitung kann ein Inline-Betrieb mit periodischer oder kontinuierlicher Messung realisiert werden.

Die Messeinheit kann in der Behandlungseinheit beispielsweise als separate Einheit oder als Bestandteil der Wasseraufbereitungsanlage verbaut sein.

Weiterhin betrifft die Erfindung ein Verfahren zur Bestimmung der Konzentration eines Keimreduktionsmittels im Behandlungswasser einer medizinischen, insbesondere zahnärztlichen Behandlungseinheit, wie in Anspruch 9 definiert. Verschiedene Ausführungsformen von Behandlungseinheit, Keimreduktionsmittel, Messeinheit, etc. ergeben sich aus der obigen Diskussion der erfindungsgemäßen Behandlungseinheit.

Alternativ zu einer Konfiguration, in der die Messeinheit als Bestandteil einer erfindungsgemäßen Behandlungseinheit vorliegt, kann das erfindungsgemäße Verfahren auch unter Verwendung einer Messeinheit durchgeführt werden, die als ein von der Behandlungseinheit separates Gerät, etwa Handheld-Gerät oder Benchtop-Gerät vorliegt.

Weitere Einzelheiten und Vorteile der Erfindung ergeben sich aus den nachfolgend anhand der Figuren diskutieren Ausführungsbeispielen. In den Figuren zeigen:
- Figur 1:: ein wasserführendes System einer erfindungsgemäßen Behandlungseinheit in einer ersten Ausführungsform;
- Figur 2:: ein wasserführendes System einer erfindungsgemäßen Behandlungseinheit in einer zweiten Ausführungsform;
- Figur 3:: ein wasserführendes System einer erfindungsgemäßen Behandlungseinheit in einer dritten Ausführungsform;
- Figur 4: ein Spektrum einer UV-Extinktionsmessung von 2-Phenoxyethanol;
- Figur 5:: ein lineares Fitting einer Messserie einer UV-Extinktionsmessung von 2-Phenoxyethanol bei einer Wellenlänge von 216 nm; und
- Figur 6:: ein lineares Fitting einer Messserie einer UV-Extinktionsmessung von 2-Phenoxyethanol bei einer Wellenlänge von 269 nm.

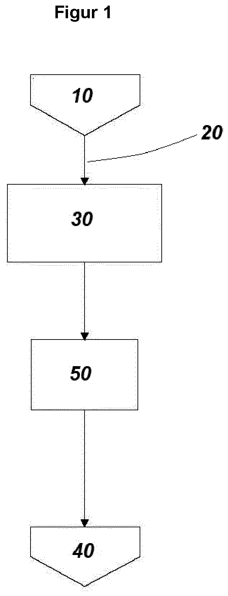

Figur 1 zeigt eine schematische Darstellung eines wasserführenden Systems einer erfindungsgemäßen Behandlungseinheit in einer ersten Ausführungsform.

Das System umfasst eine am Anfang des Systems stehende Wasserversorgung 10, z.B. einen Anschluss für eine Wasserleitung eines Gebäudes, eine Hauptleitung 20, eine an der Hauptleitung 20 angeordnete Wasseraufbereitungsanlage 30 und einen am Ende des Systems angeordneten Verbraucher 40, z.B. in Form eines Anschlusses für ein Handwerkzeug einer zahnärztlichen Behandlungseinheit.

Zwischen der Wasseraufbereitungsanlage 30 und dem Verbraucher 40 ist an der Hauptleitung 20 eine Messeinheit 50 angeordnet, die weiter unten beschrieben wird.

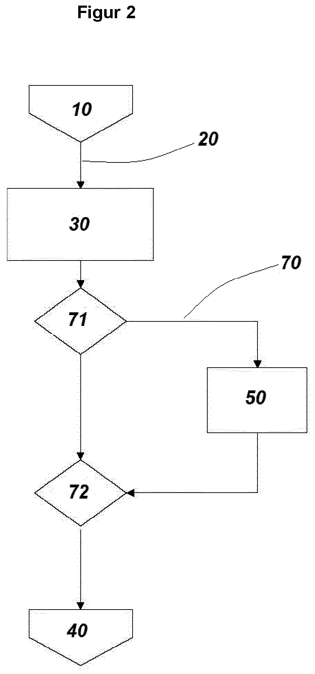

Figur 2 zeigt eine schematische Darstellung eines wasserführenden Systems einer erfindungsgemäßen Behandlungseinheit in einer alternativen Ausführungsform.

In dieser Ausführungsform ist die Messeinheit 50 nicht an der Hauptleitung 20, sondern an einer Bypass-Leitung 70 angeordnet, die im Bereich zwischen der Wasseraufbereitungsanlage 30 und dem Verbraucher 40 von der Hauptleitung 20 abzweigt und wieder in diese einmündet. Am Abzweigungspunkt und auch am Einmündungspunkt der Bypass-Leitung 70 sind jeweils Umschaltventile 71 bzw. 72 angeordnet, um einen Fluss durch die Bypass-Leitung steuern zu können.

Die mit der Wasseraufbereitungsanlage 30 und der Messeinheit 50, im Falle der Ausführungsform der Figur 2 auch mit den Ventilen 71 und 72 signalverbundene Steuereinheit 80 ist in den Figuren nicht extra dargestellt.

In der Ausführungsform der Figur 1 wie auch in der Ausführungsform der Figur 2 wird in der Wasseraufbereitungsanlage 30 ein Keimreduktionsmittel zu einem durch die Hauptleitung 20 geführten Behandlungswasser zugesetzt. Vorzugsweise handelt es sich dabei um eine Kombination aus Wasserstoffperoxid und Phenoxyethanol. Gemeinsam mit dem Keimreduktionsmittel kann ggf. auch ein UV-aktiver Beistoff in einer zum Keimreduktionsmittel proportionaler Konzentration zugegeben.

Die Messeinheit umfasst einen UV-VIS-Sensor in Kombination mit einer LED-Lichtquelle, wobei die Spektralbereiche des Sensors und der Lichtquelle auf den Absorptionsbereich des 2-Phenoxyethanols oder des Beistoffs abgestimmt sein kann. Auch im Falle einer Verwendung von z.B. HOCI, Cl₂, oder ClO₂ als Keimreduktionsmittel kann eine UV-VIS-Messung auch als direkte Messung der Konzentration des Keimreduktionsmittels, ohne Umweg über einen Beistoff, verwendet werden (nicht beansprucht).

Messbereiche könnten dabei einen Wellenlängenbereich von 200 nm bis 380 nm, vorzugsweise 240 nm bis 340 nm abdecken.

Als Photodioden für den Einsatz innerhalb einer Messeinheit einer erfindungsgemäßen Anordnung haben sich z.B. SiC-Photodioden bewährt, die einen Wellenlängenbereich von 220 nm bis 360 nm emittieren können.

Alternativ können die Messeinheiten auch ein NIR-Spektrometer umfassen, um insbesondere eine Wasserstoffperoxid-Konzentration zu bestimmen (nicht beansprucht). Die abgedeckten Wellenlängenbereiche können dabei beispielsweise von 850 bis 1800 nm reichen.

Diese Messkombination lässt sich sehr einfach und kostengünstig implementieren und kann, sowohl im Batch-Betrieb der Figur 2 also auch im Inline-Betrieb der Figur 1, eine engmaschige Messung der Konzentration des Keimreduktionsmittels realisieren. Es lässt sich also kontrollieren, ob an der Wasseraufbereitungsanlage 30 zu jedem Zeitpunkt eine ausreichende Menge an Keimreduktionsmittel in das Wasser zugegeben wurde. Diese Kontrolle erhöht die Patientensicherheit und ist zudem geeignet, den Arzt bzw. Zahnarzt rechtlich zu entlasten, sofern es beispielsweise zu einem von außen, durch Kontamination der Wasserversorgung, induzierten Legionellenbefall kommt.

Eine Weiterbildung der Erfindung umfasst eine Zwei-Punkte-Messung. Diese Weiterbildung ist apparativ aufwändiger, bringt aber zusätzliche Erkenntnisse und ermöglicht eine automatisierte Einstellung der Zugabe des Keimreduktionsmittels unter Verwendung von Messergebnissen.

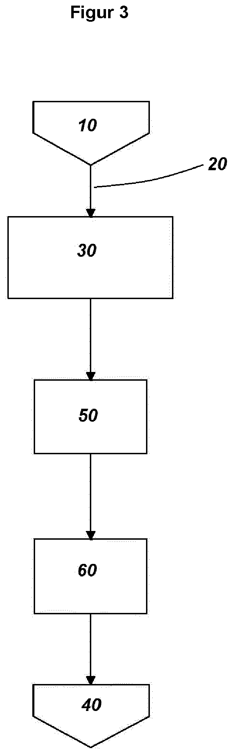

Figur 3 zeigt schematisch eine Darstellung einer Ausführungsform der Erfindung, die Gebrauch von dieser Weiterbildung macht. Grundsätzlich ähnelt der gezeigte Aufbau dem auch in Figur 1 gezeigten Aufbau, es ist aber zwischen der (ersten) Messeinheit 50 und dem Verbraucher 40, im Abstand von der Messeinheit 50, noch eine weitere Messeinheit 60 an der Hauptleitung 20 angeordnet. Zwischen den Messeinheiten 50 und 60 befindet sich ein Abschnitt des wasserführenden Systems, der Schlauchleitungen umfasst, in die Abwärme anderer Komponenten der Behandlungseinheit (z.B. Leistungselektronik, Stellmotoren) eingeleitet wird und in denen ein Bakterienwachstum daher begünstigt sein kann.

Die Messeinheiten 50 und 60 sind wie oben im Zusammenhang mit der Beschreibung der Messeinheit 50 der Figuren 1 und 2 beschrieben ausgebildet. Zudem sind in beiden Messeinheiten 50 und 60 Temperatursensoren zur Messung der Wassertemperatur vorhanden.

Die Zwei-Punkte-Messung kann somit nicht nur nachweisen, dass an der Wasseraufbereitungsanlage 30 zu jedem Zeitpunkt eine ausreichende Menge an Keimreduktionsmittel in das Wasser zugegeben wurde, sondern z.B. im Falle einer Verwendung von Sensoren, die eine Messung der Wasserstoffperoxid-Konzentration erlauben auch, ob eine Reduktion des Wasserstoffperoxids auf Grund der Oxidation organischer Verbindungen zwischen den Messeinheiten 50 und 60 stattgefunden hat. Somit kann der Wert für die Konzentrationsdifferenz als Indikator für den Grad der Kontamination herangezogen werden.

Die Steuereinheit kann ausgebildet sein, unter Rückgriff auf diese Information in der Wasseraufbereitungsanlage 30 eine Anpassung der Menge des in das Wasser zugesetzten Keimreduktionsmittels vorzunehmen.

Ferner kann die Steuereinheit ausgebildet sein, die an den Messeinheiten 50 und 60 erhaltenen Temperatur- und Konzentrationswerte in einer Weise auszuwerten, dass eine temperaturbedinge Prognose von Wachstumsbedingungen von Mikroorganismen innerhalb der Behandlungseinheit erhalten wird, und unter Rückgriff auf diese Information in der Wasseraufbereitungsanlage 30 eine Anpassung der Menge des in das Wasser zugesetzten Keimreduktionsmittels vorzunehmen.

### Beispiel 1:

Nachfolgend wird ein Beispiel (nicht beansprucht) einer Konzentrationsbestimmung von 2-Phenoxyethanol anhand von UV-spektroskopischer Extinktionsmessung beschrieben.

Der Test wurde mit einem UV-Spektrophotometer UV-16000PC durchgeführt. Als Küvette wurde eine Hellma Analytics QS High Precision Cell 1 mm verwendet. Als Proben wurden wässrige Keiminhibierungsmittel umfassend eine Kombination von Wasserstoffperoxid und 2-Phenoxyethanol sowie enthaltend einen Kalkinhibitor verwendet.

Aus dem Keiminhibierungsmittel wurde eine Verdünnungsreihe mit 10 verschiedenen Konzentrationen zwischen 10 und 300 ppm hergestellt. Anschließend wurde die Extinktion dieser Proben in dem Spektrometer mit einer Auflösung von 1 nm in einem Wellenlängenbereich von zwischen 200 und 500 nm aufgezeichnet. Die 1 mm Quartz-Küvetten wurden zwischen den Messungen mit deionisiertem Wasser und Isopropanol gereinigt und getrocknet.

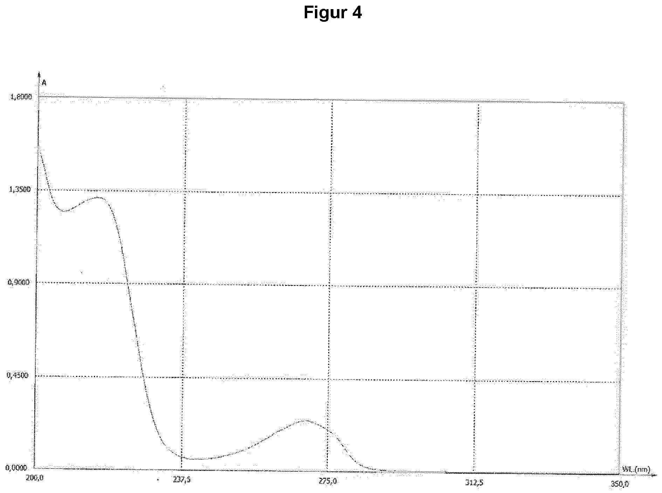

Figur 4 zeigt ein erhaltenes Spektrum der Extinktionsmessung einer Probe mit 0.025% (250 ppm) 2-Phenoxyethanol. Daraus lässt sich ableiten, dass im Extinktionsspektrum zwei charakteristische Peaks bei Wellenlängen von 216 nm und 269 nm auftreten. Die nachfolgende Tabelle 1 zeigt die gemessenen Extinktionen bei diesen Wellenlängen.

**Tabelle 1**

| Konzentration 2-Phenoxyethanol [ppm] | Extinktion bei 216 nm | Extinktion bei 269 nm |
|---|---|---|
| 10 | 0.0622 | 0.0138 |
| 20 | 0.1138 | 0.0216 |
| 30 | 0.1723 | 0.0323 |
| 40 | 0.2178 | 0.0408 |
| 50 | 0.2741 | 0.0558 |
| 100 | 0.5352 | 0.0990 |
| 150 | 0.9162 | 0.1507 |
| 200 | 1.0707 | 0.1984 |
| 250 | 1.3152 | 0.2447 |
| 300 | 1.5582 | 0.2921 |

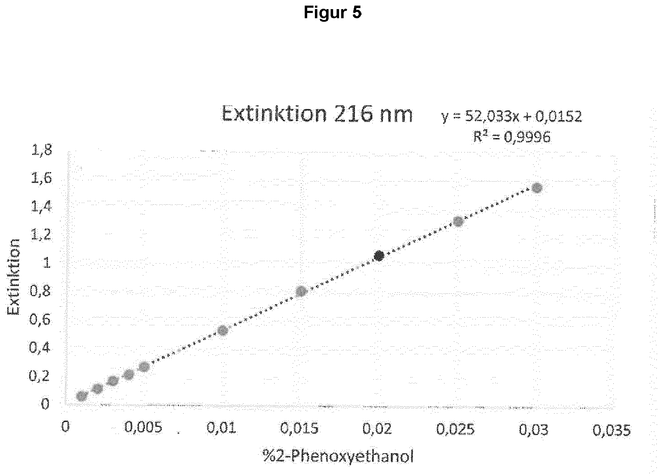

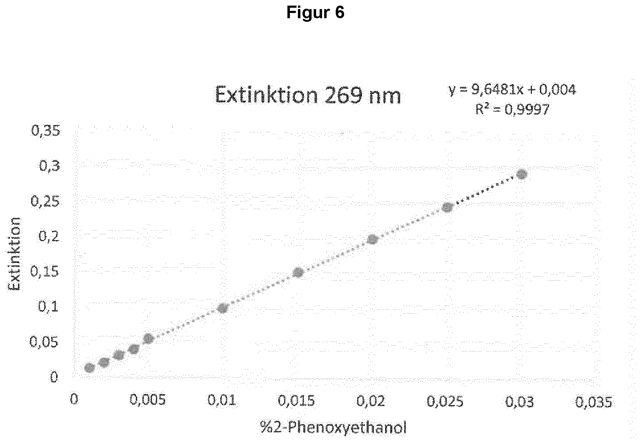

Figuren 5 und 6 zeigen ein lineares Fitting der zwei Messserien. Es ergibt sich ein linearer Zusammenhang mit einem Bestimmtheitsmaß (R²-Wert) von 0.9996 bei einer Wellenlänge von 216 nm und 0.9997 bei einer Wellenlänge von 269 nm. Um den Qualitätsanforderungen für eine hier in Rede stehende Messung zu genügen, sollte das Bestimmtheitsmaß einen Wert von 0.9990 übersteigen, was in beiden Fällen der Fall ist.

## Patentansprüche

1. Medizinische, insbesondere zahnärztliche Behandlungseinheit mit einem wasserführenden System (10, 20, 70), einer Steuereinheit und einer Wasseraufbereitungsanlage (30) zum Zusatz eines Keimreduktionsmittels in das Behandlungswasser, das in dem System (10, 20, 70) geführt wird, wobei die Behandlungseinheit ferner eine im wasserführenden System (10, 20, 70) stromabwärts der Wasseraufbereitungsanlage (30) angeordnete Messeinheit (50) zur Bestimmung der Konzentration des Keimreduktionsmittels im Behandlungswasser ausweist,
**dadurch gekennzeichnet,**
**dass** das Keimreduktionsmittel einen detektierbaren Beistoff in einer zum Keimreduktionsmittel proportionalen Menge aufweist, der selbst nicht keimreduzierend wirkt, und dass die Messeinheit (50) ausgebildet ist, die Konzentration dieses Beistoffs im Behandlungswasser zu bestimmen.

2. Behandlungseinheit nach Anspruch 1, **dadurch gekennzeichnet, dass** es sich bei dem Keimreduktionsmittel um Ozon oder eine wässrige Lösung enthaltend Wasserstoffperoxid, ein Silbersalz wie z.B. Silbernitrat, 2-Phenoxyethanol, Chlor, Hypochlorige Säure, ein Hypochlorit, ein Amin, eine quaternäre Amoniumverbindung, Iod oder eine Kombination handelt, vorzugsweise um Wasserstoffperoxid, ein Silbersalz und/oder 2-Phenoxyethanol.

3. Behandlungseinheit nach einem der vorhergehenden Ansprüche, **dadurch gekennzeichnet, dass** die Messeinheit (50) ein UV-VIS-Spektrometer zur Transmissions- oder Extinktionsmessung und/oder ein NIR-Spektrometer aufweist.

4. Behandlungseinheit nach Anspruch 3, **dadurch gekennzeichnet, dass** die Messstrecke zwischen 0.5 mm und 20 mm, vorzugsweise zwischen 2 mm und 10 mm beträgt.

5. Behandlungseinheit nach einem der vorhergehenden Ansprüche, **dadurch gekennzeichnet, dass** die Messeinheit (50) ein einen Temperatursensor und/oder eine Temperiereinheit und/oder einen pH-Sensor und/oder einen elektrochemischen Sensor umfasst.

6. Behandlungseinheit nach einem der vorhergehenden Ansprüche, **dadurch gekennzeichnet, dass** die Steuereinheit ausgebildet ist, die Zusatzmenge der Keimreduktionsmittel in der Wasseraufbereitungsanlage (30) auf der Grundlage der Messergebnisse der Messeinheit (50) automatisch zu verändern.

7. Behandlungseinheit nach einem der vorhergehenden Ansprüche, **dadurch gekennzeichnet, dass** die Behandlungseinheit zwei im wasserführenden System (10, 20, 70) hintereinander geschaltete Messeinheiten (50, 60) aufweisen, um einen etwaigen Konzentrationsabfall des Keimreduktionsmittels in einem dazwischenliegenden Abschnitt des wasserführenden Systems (10, 20, 70) messen zu können.

8. Behandlungseinheit nach einem der vorhergehenden Ansprüche, **dadurch gekennzeichnet, dass** die Behandlungseinheit eine mit der Steuereinheit signalverbundene Leseeinrichtung zum Einlesen von Codes, insbesondere von RFID- oder QR-Codes aufweist, mit denen Behälter von Keimreduktionsmitteln versehen sein können, und dass die Steuereinheit ausgebildet ist, anhand der eingelesenen Codes das eingesetzte Keimreduktionsmittel und dessen Konzentration zu bestimmen und darauf basierend gegebenenfalls eine automatische Kalibration der Sensoren und/oder eine automatische Einordnung der Messergebnisse vornehmen.

9. Verfahren zur Bestimmung der Konzentration eines Keimreduktionsmittels im Behandlungswasser einer medizinischen, insbesondere zahnärztlichen Behandlungseinheit nach einem der Ansprüchen 1 bis 8, wobei die Behandlungseinheit ein wasserführendes System (10, 20, 70) und eine Wasseraufbereitungsanlage (30) zum Zusatz des Keimreduktionsmittels, sowie zum Zusatz eines detektierbaren Beistoff in einer zum Keimreduktionsmittel proportionalen Menge, der selbst nicht keimreduzierend wirkt, in das Behandlungswasser aufweist, das in dem System (10, 20, 70) geführt wird, wobei die Messung anhand einer Messeinheit (50), welche ausgebildet ist, die Konzentration dieses Beistoffs im Behandlungswasser zu bestimmen, stromabwärts der Wasseraufbereitungsanlage (30) stattfindet.

10. Verfahren nach Anspruch 9, dass es sich bei der Behandlungseinheit um eine Behandlungseinheit nach einem der Ansprüche 1 bis 8 handelt, oder dass die Messeinheit (50) als ein von der Behandlungseinheit separates Gerät vorliegt.

## Claims

1. Medical, in particular dental treatment unit, comprising a water leading system (10, 20, 70), a control unit and a water treatment unit (30) for the addition of an antimicrobial agent to treatment water contained in the water leading system (10, 20, 70), wherein the treatment unit further comprises a measuring unit (50) arranged in the water leading system (10, 20, 70) downstream of the water treatment unit (30) for determining the concentration of the antimicrobial agent in the treatment water,
**characterized in that**
the antimicrobial agent comprises a detectable co-formulant, which does not have antimicrobial activity itself, in an amount proportional to the antimicrobial agent, and that the measurement unit is configured to determine the concentration of this co-formulant in the treatment water.

2. Treatment unit according to claim 1, **characterized in that** the antimicrobial agent is ozone or an aqueous solution containing hydrogen peroxide, a silver salt like silver nitrate, 2-phenoxyethanol, chlorine, hypochloric acid, a hypochlorite, an amine, a quaternary ammonium compound, iodine, or a combination thereof, preferably hydrogen peroxide, a silver salt, and/or 2-phenoxyethanol.

3. Treatment unit according to any one of the preceding claims, **characterized in that** the measurement unit (50) comprises an UV-vis spectrometer configured for transmission or absorbance measurement, and/or a NIR spectrometer.

4. Treatment unit according to claim 3, **characterized in that** the measurement path is 0,5 mm to 20 mm, preferably between 2 mm to 10 mm.

5. Treatment unit according to any one of the preceding claims, **characterized in that** the measurement unit (50) comprises a temperature sensor and/or a tempering unit and/or a pH sensor and/or an electrochemical sensor.

6. Treatment unit according to any one of the preceding claims, **characterized in that** the control unit is configured to automatically change the addition amount of the antimicrobial agents in the water treatment system (30) on the basis of the measuring results of the measuring unit (50).

7. Treatment unit according to any one of the preceding claims, **characterized in that** the treatment unit comprises two measuring units (50, 60) connected in series in the water leading system (10, 20, 70) in order to be able to measure a possible dop in concentration of the antimicrobial agent in a section of the water leading system (10, 20, 70) located therebetween.

8. Treatment unit according to any one of the preceding claims, **characterized in that** the treatment unit comprises a reader which is in signal connection with the control unit, for reading codes, in particular RFID or QR codes which may be attached to containers of the antimicrobial agents, and **in that** the control unit is configured to determine the antimicrobial agent used and its concentration on the basis of the read code and, on this basis, carry out an automatic calibration of the sensors and/or an automatic evaluation of the measurement results, if required.

9. Method of determining a concentration of an antimicrobial agent in treatment water of a medical, in particular dental treatment unit in accordance with any one of claims 1 to 8, wherein the treatment unit comprises a water leading system (10, 20, 70) and a water treatment unit (30) for adding the antimicrobial agent and for adding a detectable co-formulant, which itself has no antimicrobial effect, in an amount proportional to the antimicrobial agent to the treatment water, which is contained in the system (10, 20, 70), and wherein the measuring is effected by means of a measuring unit (50) which is configured to determine the concentration of this co-formulant in the treatment water downstream of the water treatment unit (30).

10. Method according to claim 9, **characterized in that** the treatment unit is a treatment unit according to any one of claims 1 to 8, or **in that** the measuring unit (50) is provided as a device separate from the treatment unit.

## Revendications

1. Unité de traitement médicale, en particulier dentaire, dotée d'un système à circulation d'eau (10, 20, 70), d'une unité de commande et d'une installation de traitement de l'eau (30) destinée à ajouter un agent réducteur de germination dans l'eau de traitement qui circule dans le système (10, 20, 70), l'unité de traitement présentant en outre une unité de mesure (50) disposée dans le système à circulation d'eau (10, 20, 70), en aval de l'installation de traitement de l'eau (30), pour déterminer la concentration de l'agent réducteur de germination dans l'eau de traitement,
**caractérisée en ce que**
l'agent réducteur de germination présente, dans une quantité proportionnelle à l'agent réducteur de germination, un coformulant apte à être détecté, qui n'a lui-même aucune action réductrice de germination, et **en ce que** l'unité de mesure (50) est conçue pour déterminer la concentration de ce coformulant dans l'eau de traitement.

2. Unité de traitement selon la revendication 1, **caractérisée en ce que** l'agent réducteur de germination est de l'ozone ou une solution aqueuse contenant du peroxyde d'hydrogène, un sel d'argent, tel que par exemple du nitrate d'argent, du 2-phénoxyéthanol, du chlore, de l'acide hypochloreux, un hypochlorite, une amine, un composé ammonium quaternaire, de l'iode ou une combinaison, de préférence du peroxyde d'hydrogène, un sel d'argent et/ou du 2-phénoxyéthanol.

3. Unité de traitement selon l'une des revendications précédentes, **caractérisée en ce que** l'unité de mesure (50) présente un spectromètre UV/visible pour la mesure de la transmission ou de l'extinction et/ou un spectromètre NIR.

4. Unité de traitement selon la revendication 3, **caractérisée en ce que** la distance de mesure est comprise entre 0,5 mm et 20 mm, de préférence entre 2 mm et 10 mm.

5. Unité de traitement selon l'une des revendications précédentes, **caractérisée en ce que** l'unité de mesure (50) comprend un capteur de température et/ou une unité de contrôle de température et/ou un capteur de pH et/ou un capteur électrochimique.

6. Unité de traitement selon l'une des revendications précédentes, **caractérisée en ce que** l'unité de commande est conçue pour modifier automatiquement la quantité d'agent réducteur de germination ajoutée dans l'installation de traitement de l'eau (30) en fonction des résultats de mesure de l'unité de mesure (50).

7. Unité de traitement selon l'une des revendications précédentes, **caractérisée en ce que** l'unité de traitement présente deux unités de mesure (50, 60) montées l'une derrière l'autre dans le système à circulation d'eau (10, 20, 70) pour pouvoir mesurer une baisse de concentration éventuelle de l'agent réducteur de germination dans une section intermédiaire du système à circulation d'eau (10, 20, 70).

8. Unité de traitement selon l'une des revendications précédentes, **caractérisée en ce que** l'unité de traitement présente, relié par signal à l'unité de commande, un dispositif de lecture destiné à la lecture de codes, en particulier de codes RFID ou QR, dont peuvent être pourvus des contenants d'agent réducteur de germination, et **en ce que** l'unité de commande est conçue pour, à l'aide des codes lus, déterminer l'agent réducteur de germination utilisé et sa concentration et, sur cette base, effectuer le cas échéant un étalonnage automatique des capteurs et/ou une classification automatique des résultats de mesure.

9. Procédé de détermination de la concentration d'un agent réducteur de germination dans l'eau de traitement d'une unité de traitement médicale, en particulier dentaire, selon l'une des revendications 1 à 8, l'unité de traitement présentant un système à circulation d'eau (10, 20, 70) et une installation de traitement de l'eau (30) destinée à ajouter l'agent réducteur de germination, ainsi qu'à ajouter, dans une quantité proportionnelle à l'agent réducteur de germination, un coformulant apte à être détecté qui n'a lui-même aucune action réductrice de germination, dans l'eau de traitement qui circule dans le système (10, 20, 70), la mesure ayant lieu en aval de l'installation de traitement de l'eau (30), à l'aide d'une unité de mesure (50) qui est conçue pour déterminer la concentration de ce coformulant dans l'eau de traitement.

10. Procédé selon la revendication 9, **caractérisé en ce que** l'unité de traitement est une unité de traitement selon l'une des revendications 1 à 8, ou **en ce que** l'unité de mesure (50) se présente sous la forme d'un appareil séparé de l'unité de traitement.
